# EUROPÄISCHE PATENTSCHRIFT

(11) **EP 3 846 869 B1**
(45) Veröffentlichungstag und Bekanntmachung des Hinweises auf die Patenterteilung: **26.07.2023**
(21) Anmeldenummer: 19774054.1
(22) Anmeldetag: 02.09.2019
(51) Int. Cl.: A61L 31/16, A61L 15/46, A61K 9/00

(54) **FLÄCHENPRODUKT ZUR MIKROBIOLOGISCHEN SANIERUNG DES UROGENITALTRAKTS**
FLAT PRODUCT FOR MICROBIOLOGICAL SANITATION OF THE UROGENITAL TRACT
PRODUIT PLAT POUR LA DÉSINFECTION MICROBIOLOGIQUE DU TRACTUS UROGÉNITAL

(30) Priorität: 04.09.2018 WO PCT/EP2018/073776
(43) Veröffentlichungstag der Anmeldung: 14.07.2021
(73) Patentinhaber: Tecuro Medical GmbH, 6003 Luzern (CH)
(72) Erfinder: HENGSBERGER, Corinna, 79650 Schopfheim (DE); VON STETTEN, Otto, 52072 Aachen (DE); RIES, Gerd, 47259 Duisburg (DE)
(74) Vertreter: Rentsch Partner AG
(86) Internationale Anmeldenummer: PCT/EP2019/073317
(87) Internationale Veröffentlichungsnummer: WO 2020/048910

(56) Entgegenhaltungen:
- WO-A1-2015/154746
- WO-A1-2017/055135

## Beschreibung

### Technisches Gebiet

Die vorliegende Erfindung bezieht sich auf das Gebiet der Hygienetechnik im Urogenitalbereich. Insbesondere bezieht sich die Erfindung auf ein Flächenprodukt zur Behandlung des Urogenitaltrakts, sowie zur Anwendung in der Therapie oder der Prophylaxe von beispielsweise Harnwegsentzündungen und anderen Infektionskrankheiten. Des Weiteren bezieht sich die vorliegende Erfindung auf ein Herstellungsverfahren eines Flächenprodukts zur Behandlung des Urogenitaltrakts.

### Stand der Technik

Nieren- und Harnwegsentzündungen sind häufige Infektionskrankheiten, wobei wegen der anatomischen Verhältnisse Frauen wesentlich häufiger betroffen sind als Männer. Etwa 10% der Frauen in den USA und Europa leiden an rezidivierenden Infekten mit drei und mehr Episoden im Jahr. Ursache sind hauptsächlich *E. coli* Bakterien, aber auch Klebsiellen, Proteus oder Staphylokokken, die aus dem eigenen Darm stammen. Sie wandern aus dem Anus über die Haut in die Urethra und schliesslich in die Blase und von dort ins Nierenbecken. Selbst nach einer überstandenen Entzündung können sich am Eingang der Urethra Nischen mit überlebenden Bakterien bilden, die zum Rezidiv führen können.

Zur Therapie werden oft systemisch applizierte Antibiotika eingesetzt, die aber bei der notwendig häufigen Anwendung wegen der entstehenden Resistenzen der Bakterien unwirksam werden, sowie unerwünschte Nebenwirkungen aufweisen können. Die derzeit populäre Phytotherapie, hauptsächlich basierend auf Preiselbeeren oder Cranberry, zeigte in Studien variable Ergebnisse und konnte bisher nicht validiert werden.

Neuere Ansätze beschreiben Methoden, die beteiligten Bakterien an der Wanderung zu hindern, bzw. diese zu eliminieren, bevor sie die Urethra und damit die Blase oder die Nieren erreichen können.

Die US-Patentanmeldung US 2002/0115976 schlägt eine Hygienebinde vor, die den Anusbereich, den Damm und den Genitalbereich der Frau abdeckt. Im zentralen Dammbereich ist eine mechanische Hürde vorgesehen, die quer auf der Binde verläuft. Diese Hürde kann anti-mikrobielle Substanzen aufweisen, sodass die Bakterien angeblich nicht vom Anus zum Genitalbereich wandern können. Es wird ausdrücklich gefordert, dass die antimikrobiellen Substanzen den genitalen Bereich nicht erreichen. Mikroorganismen werden allerdings nicht durch eine mechanische Hürde aufgehalten, selbst wenn diese anti-mikrobielle Substanzen enthält, da sich Mikroorganismen alle möglichen Wege zu ihrem Ziel suchen.

Die WO 201 5/1 54746 schlägt eine Abdeckung für den urogenitalen Bereich, zumindest im Bereich der Scham, der Vagina und der Urethra vor, die im Wesentlichen vollständig mit einer antiseptischen Substanz gegen die Einwanderung von Bakterien in den urogenitalen Trakt präpariert ist.

### Darstellung der Erfindung

Durch die Verwendung von antimikrobiell und antiseptisch wirksamen Mitteln in Abdeckungen für den urogenitalen Bereich, kommen die empfindlichen Schleimhäute des Urogenitaltrakts in engen Kontakt mit diesen Substanzen. Da solche Abdeckungen erfahrungsgemäss über eine relative lange Zeit angewendet und getragen werden, stellen Hautreizungen, Abschürfungen und Unverträglichkeiten bis hin zur Ausbildung von Allergien ein signifikantes Problem solcher Abdeckungen dar. Der Fokus der Forschung lag lange Zeit auf der Suche nach einem möglichen Ersatz für antibiotische Mittel, um die Bildung von Resistenzen zu verhindern. Das Augenmerk lag hierbei besonders darin, Substanzen zu identifizieren, welche die betreffenden Bakterien mit gleicher oder zumindest annähernd gleicher Wirksamkeit bekämpfen können. Dabei wurde allerdings die Hautverträglichkeit solcher Ersatzsubtanzen ausser Acht gelassen. Bekannte Antiseptika sind häufig aggressiv gegenüber der Haut und insbesondere gegenüber den Schleimhautzellen. Zwar können Hygienebinden mit Feuchtigkeitsspendern versehen werden, um eine erhöhte Hautreibung der textilen Lagen zu vermeiden, jedoch haben diese keine Auswirkung auf die aggressiv wirkenden Antiseptika.

Es ist daher die Aufgabe der Erfindung, den Stand der Technik im Bereich der Hygienetechnik im Urogenitalbereich zu verbessern. In vorteilhaften Ausführungsformen kann durch die vorliegende Erfindung eine Vorrichtung zur Behandlung des Urogenitaltrakts bereitgestellt werden, welche eine verbesserte Hautverträglichkeit und bevorzugt eine ausreichende Wirksamkeit gegenüber Bakterien aufweist. In weiteren vorteilhaften Ausführungsformen können zuverlässig Infektionen des Urogenitaltrakts verhindert und/oder behandelt werden.

Eine weitere Aufgabe der Erfindung ist es den Stand der Technik im Bereich der Herstellungsverfahren von Vorrichtungen in der Hygienetechnik zu verbessern. Gemäss vorteilhaften Ausführungsformen wird ein kostengünstiges und/oder effizientes Herstellungsverfahren für eine Vorrichtung zur Behandlung des Urogenitaltrakts bereitgestellt.

Mindestens eine dieser Aufgaben wird in allgemeiner Weise durch den Gegenstand der unabhängigen Patentansprüche gelöst. Weitere vorteilhafte Ausführungsformen ergeben sich jeweils aus den abhängigen Patentansprüchen, sowie der Offenbarung insgesamt.

In einem ersten Aspekt betrifft die Erfindung ein Flächenprodukt zur Behandlung des Urogenitaltrakts mit einer proximalen Fläche und einer distalen Fläche. Das Flächenprodukt umfasst mindestens eine textile Lage. Die proximale Fläche bildet im operativen Zustand die der Haut zugewandte Fläche des Flächenprodukts und die distale Fläche die im operativen Zustand der Haut abgewandte Fläche des Flächenprodukts. Die proximale Fläche ist mit einem antimikrobiell und/oder antiseptisch wirksamen Mittel beschichtet, wobei das Flächenprodukt im nachfolgend beschriebenen Zytotoxizitätstest eine Vitalität von >80% nach 18 Stunden (h) Inkubationszeit aufweist. Des Weiteren weist das Flächenprodukt im nachfolgend beschriebenen PI-MIA Test eine Wirksamkeit von >80% auf. Das Flächenprodukt ist dadurch gekennzeichnet, dass das antimikrobiell und/oder antiseptisch wirksame Mittel Weinsäure in einer Konzentration von 2 bis 4 g/L enthält.

In einer Ausführungsform der Erfindung weist die proximale Fläche des Flächenprodukts zur Behandlung des Urogenitaltrakts in der Mitte in Längsrichtung eine Steppnaht auf. In Längsrichtung bedeutet im Sinne der Erfindung, dass diese im operativen Zustand in Richtung der Schamlippen verläuft und nicht quer dazu. In Ausführungsformen mit mehreren textilen Lagen kann die Steppnaht auch zum Verbinden von mindestens zwei textilen Lagen ausgebildet sein. Eine Steppnaht erleichtert im Allgemeinen das Knicken des Flächenprodukts, vergrössert die Kontaktfläche zur Haut und verhindert ein Verrutschen des erfindungsgemässen Flächenprodukts während des Tragens. Typischerweise beträgt die Länge der Naht im Wesentlichen 2/3 oder mehr der Länge des Flächenprodukts.

In weiteren Ausführungsformen ist die distale Fläche hydrophobiert. Diese kann dazu entweder mit einem hydrophoben Material wie beispielsweise Fluorcarbon beschichtet sein oder aus einem solchem Material bestehen. Solche Ausführungsformen erlauben es allfällige Leckagen zu verhindern und stellen zudem sicher, dass das antimikrobiell und/oder antiseptisch wirksame Mittel nicht aus dem Flächenprodukt austritt und im operativen Zustand nach aussen verdampft oder an die Kleidung abgegeben wird.

In einer weiteren Ausführungsform umfasst mindestens eine textile Lage ein hautkompatibles Textil, beispielsweise aus hautkompatiblen Gewebe, Gewirke oder non-Wovens. In Ausführungsformen mit mehreren textilen Lagen, können diese unterschiedliche Materialien umfassen, wobei dann typischerweise zumindest die proximale Fläche ein hautkompatibles Material umfasst. Hautkompatible Materialien sind bevorzugt Gewebe, Gewirke und Non-Wovens, beispielswiese Baumwolle, Polypropylen, Polyester, Polyethylen, Polyamid, Nylon, etc. Die Oberfläche der textilen Lagen, insbesondere des proximalen Fläche, kann gegebenenfalls strukturiert ausgebildet sein. Durch die Verwendung von solchen hautkompatiblen Materialien kann vorzugsweise der Tragekomfort erhöht und Hautreizungen verhindert werden.

In einer typischen Ausführungsform ist die proximale Fläche in Streifen oder punktuell zur besseren Fixierung auf der Haut mit bioadhäsiven und/oder mucoadhäsiven Mitteln beschichtet. Solche Mittel dienen dazu, das Flächenprodukt eng auf der Haut zu platzieren und verbessern so die Wirksamkeit gegenüber Bakterien. Zusätzlich wird durch die verbesserte Haftung des Flächenprodukts die Reibung reduziert, was den Tragekomfort erhöht. Als bioadhäsive und/oder mucoadhäsive Mittel können beispielsweise Carbomere, Poloxamere, Polyacrylate und Derivative, Chitosane und Chitosanderivate, Alginatpolyethylenglycolacrylate, Thiomere (z.B. Polycarbophil-cystein, etc.), Lectine, sowie deren Mischungen eingesetzt werden.

In einer bevorzugten Ausführungsform umfasst das antimikrobiell und/oder antiseptisch wirksame Mittel Tenside, quaternäre Ammoniumverbindungen, Biguanide, organischen Säuren oder Salze davon, Metallionen wie Kupfer, Zink, Eisen, Silber, Ruthenium, Rhodium, Jodverbindungen, Aldehyde und Mischungen derselben.

Das antimikrobiell und/oder antiseptisch wirksame Mittel umfasst Weinsäure oder ein Salz davon in einer Konzentration von 2 bis 4 g/L. Insbesondere kann die Weinsäure in einer maximalen Konzentration von 3 g/L, beispielsweise 1 bis 3 g/L vorliegen. Es hat sich gezeigt, dass sich Weinsäure in diesem Konzentrationsbereich als vorteilhaft für auf die Zellvitalität gemäss dem hier beschriebenen Zytotoxizitätstest auswirkt.

Typischerweise umfasst das antimikrobiell und/oder antiseptisch wirksame Mittel mindestens ein Vitamin, insbesondere Vitamin A, B, C, E, mindestens ein Metallion der achten, elften oder zwölften Gruppe des Periodensystems nach IUPAC, z.B. Eisen, Kupfer oder Zink, mindestens eine aliphatische oder aromatische organische Säure oder ein Derivat derselben und mindestens ein Tensid. Optional umfasst das antimikrobiell und/oder antiseptisch wirksame Mittel zusätzlich Basen und/oder Puffersysteme zur Einstellung des pH-Werts. Ein Flächenprodukt mit einem solchen Mittel erfüllt besonders zufriedenstellend sowohl die Erfordernisse der Hautverträglichkeit, als auch der Wirksamkeit. Als besonders hautverträglich haben sich Ausführungsformen herausgestellt, welche zusätzlich Basen und/oder Puffersysteme beinhalten. Überraschenderweise führt die geringere Azidität des Mittels nicht zu einem signifikanten Rückgang der antibakteriellen, antimikrobiellen oder antiseptischen Wirksamkeit.

Die Vitamine können in ihren gängigen oder ionischen Formen eingesetzt werden. Besonders bevorzugt wird Vitamin C, Riboflavin und/oder Niacin verwendet. Die Konzentration der Vitamine kann dabei zwischen 0,5 und 1,5 g/L liegen.

Als Metallionquelle können gängige Salze der jeweiligen Metallionen dienen, beispielsweise Halogenide, Sulfate, Nitrate, Sulfite oder Phosphate. Bevorzugte Metallionen sind Kupfer, Eisen und Zink. Die Konzentrationen bewegen sich zwischen 0,5 und 1,5 g/L, vorzugsweise 0,75 und 1,25 g/L. Diese Konzentrationen haben sich als besonders vorteilhaft erwiesen, da diese hoch genug sind um eine ausreichende Wirksamkeit gegenüber Bakterien zu gewährleisten, sich gleichzeitig aber auch nicht negativ auf die Hautverträglichkeit auswirken.

Derivate von organischen Säuren, welche ebenfalls als organische Säuren verwendet werden können, können insbesondere deren deprotonierte Formen umfassen. Diese können entweder aus den jeweiligen Salzen, z.B. Ammonium, Natrium, Kalium, Magnesium, Calcium, Lithium, etc. erhalten werden, oder durch Deprotonierung der Säuren mit den optional enthaltenen Basen. Des Weiteren umfassen solche Derivate Ester- oder Amidverbindungen. Typische Beispiele sind hierbei Ameisensäure, Essigsäure, Bromessigsäure, Glykolsäure, Propionsäure, Glyoxylsäure, Milchsäure, Zitronensäure, Weinsäure, Malonsäure, Maleinsäure, Fumarsäure, Pyrrolidoncarbonsäure, Sorbinsäure, Undecylensäure, Undecinsäure, Benzoesäure, Hydroxybenzoesäure, Salicylsäure, Dehydracetsäure, 4-Hydroxybenzoesäureester, Dimethylcarbonat, Chloracetamid, 2-Chlor-N-(hydroxymethyl)acetamid, Salicylanilid.

Die Tenside werden aus der Gruppe der anionischen, nichtionischen, amphoteren oder kationischen Tenside ausgewählt. Dies sind z.B. Alkylethersulfate, Alkyl- und/oder Arylsulfonate, Alkylsulfate, Olefinsulfonate, Amphotenside, Betaine, Alkylamidoalkylamine, alkylsubstituierte Aminosäuren und /oder Iminosäuren, acylierte Aminosäuren, Zuckertenside, quartäre Ammoniumverbindungen, etc. Die Tenside werden entweder einzeln oder in geeigneten Mischungen untereinander verwendet. Vorzugsweise werden anionische Tenside verwendet, besonders bevorzugt Laurylsulfate, insbesondere Natriumlaurylsufat. Die typischen Mengen bewegen sich zwischen 1,5 und 10 g/L, vorzugsweise 2,0 und 8 g/L, besonders bevorzugt 2,5 und 6,5 g/L.

Typische Puffersysteme sind beispielsweise Phosphat-, Acetat-, Zitrat-, Tris(hydroxymethyl)aminomethan- (Tris), Succinat- Carbonat,- Borat- Oxalat-, Glycin-, oder Tartratpuffer.

In einer weiteren Ausführungsform umfasst das antimikrobiell und/oder antiseptisch wirksame Mittel mindestens einen Gelbildner. Solche Gelbildner sind besonders vorteilhaft, da sich überraschenderweise gezeigt hat, dass über deren Konzentration oder über die resultierende Schichtdicke die Freisetzung des wirksamen Mittels gesteuert werden kann, sodass über eine ausreichend lange Zeit eine genügende Menge des wirksamen Mittels zur Verfügung steht, ohne dass das Flächenprodukt vor der Anwendung mit dem Mittel überladen werden müsste. Hierdurch kann die Dauer der Wirksamkeit signifikant verbessert werden.

In bevorzugten Ausführungsformen beträgt die Konzentration des Gelbildners im antimikrobiell und/oder antiseptisch wirksamen Mittel 2.5 bis 45 g/L, bevorzugt 5 bis 35 g/L, besonders bevorzugt 7.5 g/L bis 25 g/L.

Typischerweise umfasst der mindestens eine Gelbildner ein Polysaccharid, vorzugsweise Agarose, Carrageenan, Pektin, Xanthan, Alginsäure und Alginate, Siliziumdioxid, Polyvinylpyrrolidone, Polyvinylalkohole, Polymethacrylate, vorzugsweise Poly(hydroxy methacrylate), Poly(N-isopropyl acrylamide), Polyacrylsäuren oder Mischungen derselben. Hierbei sind Polysaccharide besonders bevorzugte Gelbildner, welche sich besonders vorteilhaft auf die Freisetzung des Mittels auswirken. Des Weiteren hat sich gezeigt, dass natürliche Gelbildner den vorteilhaften Effekt haben, dass das Risiko von allergischen Reaktionen noch weiter reduziert wird.

In einer bevorzugten Ausführungsform enthält das antimikrobiell und/oder antiseptisch wirksame Mittel als Vitamin Ascorbinsäure, als Metallionen Kupfer, als organische Säure Zitronen- und/oder Weinsäure oder ein Salz davon, als Tensid Laurylsulfate, insbesondere Natriumlaurylsuflat, und optional als Gelbildner Agarose oder Carrageenan.

In einigen bevorzugten Ausführungsformen enthält das antimikrobiell und/oder antiseptisch wirksame Mittel als Vitamin Ascorbinsäure, als Metallionen Kupfer, Eisen oder Zink, als organische Säure Zitronen- und/oder Weinsäure oder ein Salz davon, als Tensid Laurylsulfate, insbesondere Natriumlaurylsuflat, und optional als Gelbildner Agarose oder Carrageenan. Die Konzentration der Weinsäure im antimikrobiell und/oder antiseptisch wirksamen Mittel beträgt 2 bis 4 g/L.

In einer weiteren Ausführungsform beträgt die Konzentration des mindestens einen Vitamins 0.5 bis 1.5 g/L; und /oder
der mindestens einen aliphatischen oder aromatischen Säure 2 bis 6.5 g/L; und/oder
der Metallionen 0.5 bis 1.5 g/L; und/oder
der Tenside 1.5 bis 10 g/L, bevorzugt 2 bis 8 g/L, besonders bevorzugt 2.5 bis 6.6 g/L; und/oder
des mindestens einen Gelbildners 2.5 bis 45 g/L, bevorzugt 5 bis 35 g/L, besonders bevorzugt 7.5 g/L bis 25 g/L.

In bevorzugten Ausführungsformen beträgt die Konzentration des mindestens einen Vitamins 0.5 bis 1.5 g/L; und
der mindestens einen aliphatischen oder aromatischen Säure 2 bis 6.5 g/L; und
der Metallionen 0.5 bis 1.5 g/L; und
der Tenside 1.5 bis 10 g/L, bevorzugt 2 bis 8 g/L, besonders bevorzugt 2.5 bis 6.6 g/L.

In weiteren Ausführungsformen beträgt die Konzentration des mindestens einen Vitamins 0.5 bis 1.5 g/L; und
der mindestens einen aliphatischen oder aromatischen Säure 2 bis 6.5 g/L; und
der Metallionen 0.5 bis 1.5 g/L; und
der Tenside 1.5 bis 10 g/L, bevorzugt 2 bis 8 g/L, besonders bevorzugt 2.5 bis 6.6 g/L; und
des mindestens einen Gelbildners 2.5 bis 45 g/L, bevorzugt 5 bis 35 g/L, besonders bevorzugt 7.5 g/L bis 25 g/L.

In bevorzugten Ausführungsformen enthält das antimikrobiell und/oder antiseptisch wirksame Mittel als Vitamin Ascorbinsäure in einer Konzentration von 0.5 bis 1.5 g/L; und mindestens eine aliphatische oder aromatischen Säure oder ein Salz davon in einer Konzentration von 2 bis 6.5 g/L; und Metallionen in einer Konzentration von 0.5 bis 1.5 g/L; und Tenside in einer Konzentration von 1.5 bis 10 g/L, bevorzugt 2 bis 8 g/L, besonders bevorzugt 2.5 bis 6.6 g/L.

In weiteren Ausführungsformen enthält das antimikrobiell und/oder antiseptisch wirksame Mittel als Vitamin Ascorbinsäure in einer Konzentration von 0.5 bis 1.5 g/L; und mindestens eine aliphatische oder aromatischen Säure oder ein Salz davon in einer Konzentration von 2 bis 6.5 g/L; als Metallionen Kupfer, Eisen und/oder Zink in einer Konzentration von 0.5 bis 1.5 g/L; und Tenside in einer Konzentration von 1.5 bis 10 g/L, bevorzugt 2 bis 8 g/L, besonders bevorzugt 2.5 bis 6.6 g/L.

In einer weiteren Ausführungsform enthält das antimikrobiell und/oder antiseptisch wirksame Mittel als Vitamin Ascorbinsäure in einer Konzentration von 0.5 bis 1.5 g/L, als Metallionen Kupfer, Eisen und/oder Zink in einer Konzentration von 0.5 bis 1.5 g/L, als organische Säure Weinsäure in einer Konzentration von 2 bis 4 g/L, als Tensid Laurylsulfate, insbesondere Natriumlaurylsuflat, in einer Konzentration von 2 bis 8 g/L, bevorzugt 2.5 bis 6.6 g/L. Zusätzlich kann das antimikrobiell und/oder antiseptisch wirksame Mittel optional als Gelbildner Agarose oder Carrageenan in einer Konzentration Gelbildners 2.5 bis 45 g/L, bevorzugt 5 bis 35 g/L, besonders bevorzugt 7.5 g/L bis 25 g/L enthalten. Es hat sich gezeigt, dass solche Ausführungsformen sowohl eine sehr hohe Wirksamkeit und gleichzeitig eine hohe Hautverträglichkeit aufweisen.

In einigen Ausführungsformen weist die proximale Fläche des erfindungsgemässen Flächenprodukts eine Fläche von 20 bis 25 cm² auf, bevorzugt 23 cm². Typischerweise wird auf eine solche Fläche in weiteren Ausführungsformen zwischen 0.3 und 1.8 mL des antimikrobiell und/oder antiseptisch wirksamen Mittels aufgebracht. Dies entspricht einer Dotierung von 0.015 bis 0.078 mL/cm². Ein solcher Wert ist auch bei anderen Flächenwerten der proximalen Fläche eines erfindungsgemässen Flächenprodukts vorteilhaft und damit nicht auf eine Fläche von 20 bis 25 cm² beschränkt. Eine Dotierung von 0.015 bis 0.078 mL/cm² ist einerseits niedrig genug, dass das Flächenprodukt nicht von der Flüssigkeit durchsetzt ist und damit den Tragekomfort reduziert und andererseits gross genug, dass eine ausreichende und wirksame Menge zur Bekämpfung der Bakterien zur Verfügung steht.

In einer bevorzugten Ausführungsform weist das antimikrobiell und/oder antiseptisch wirksame Mittel einen pH-Wert zwischen 1.8 und 5.2, bevorzugt 2.0 bis 4.2, insbesondere 2.3 bis 3.1 auf. Hierdurch kann die Hautverträglichkeit zusätzlich erhöht und die Reizungen vermieden werden. Dieser Effekt zeigt sich erstaunlicherweise besonders deutlich bei einem pH-Wert von 2.3 bis 3.1, was signifikant unterhalb des natürlichen pH-Werts der Vaginalflora (4 bis 4.5) liegt.

In typischen Ausführungsformen weist das Flächenprodukt zur Behandlung des Urogenitaltrakts eine Form auf, die derart ausgestaltet ist, dass diese interlabial platzierbar und tragbar ist. Dem Fachmann ist es beispielsweise durch die Form verschiedener Hygienebinden bekannt, welche Formen dabei vorteilhaft sind. So kann das Flächenprodukt eine längliche Form, insbesondere mit abgerundeten Ecken, aufweisen, oder oval, rund, insbesondere kreisrund, rechteckig oder quadratisch sein. Zudem kann das erfindungsgemässe Flächenprodukt sogenannte Flügel umfassen, wie es dem Fachmann bekannt ist.

Das Flächenprodukt kann im operativen Zustand den urogenitalen Raum abdecken, zumindest im Bereich der Scham, der Vagina und der Urethra. Typischerweise kann die Form des Flächenprodukts den Gegebenheiten der Anatomie im Urogenitalraum folgen.

Bevorzugt weist das Flächenprodukt eine längliche Form auf, welche typischerweise an den Enden abgerundet ist.

In einer Ausführungsform der Erfindung weist das Flächenprodukt zur Behandlung des Urogenitaltrakts mehrere textile Lagen auf. Diese können jeweils aus verschiedenen Materialien oder auch vollständig oder teilweise aus denselben Materialien bestehen. Die mehreren Lagen sind typischerweise durch Verkleben, Stanzen, Verschweissen, Vernähen oder mithilfe von Mikro- oder Ultraschallwellen miteinander verbunden. In vorteilhaften Ausführungsformen wird die distale Fläche des Flächenprodukts von einer textilen Lage aus einem hydrophobierten Material oder einer weiteren hydrophoben Schicht gebildet. Umfasst das Flächenprodukt mehrere textile Lagen, kann mindestens eine Lage ein saugfähiges Material, wie zum Beispiel Watte oder ein anderes absorptives Material, umfassen. Diese kann beispielsweise in einem dreilagigen Aufbau die Mittellage bilden, oder in einem zweilagigen Aufbau bevorzugt mit einem hydrophoben Material beschichtet sein.

Es ist zudem möglich, dass ein erfindungsgemässes Flächenprodukt direkt auf eine handelsübliche Hygienebinde in geeigneter Form aufgebracht wird, beispielsweise durch Kleben, Nähen oder Stanzen.

Ein solches Flächenprodukt kann in der therapeutischen oder prophylaktischen Behandlung von Infektionskrankheiten des Urogenitalbereichs verwendet werden. Besonders vorteilhaft ist ein erfindungsgemässes Flächenprodukt zur Anwendung in der Therapie oder Prophylaxe von Blasen-, Nieren-, und Harnwegsentzündungen.

Ein weiterer Aspekt der Erfindung betrifft ein Verfahren zur Herstellung eines erfindungsgemässen Flächenprodukts zur Behandlung des Urogenitaltrakts, umfassend die Schritte: (a) Bereitstellen mindestens einer textilen Lage; (b) Beschichtung einer proximalen Fläche mit einem antimikrobiell und/oder antiseptisch wirksamen Mittel, sodass das Flächenprodukt im hier beschriebenen Zytotoxizitätstest eine Vitalität von > 80% nach 18 h Inkubationszeit und im hier beschriebenen PI-MIA Test eine Wirksamkeit von > 80 % aufweist, dadurch gekennzeichnet, dass das antimikrobiell und/oder antiseptisch wirksame Mittel Weinsäure in einer Konzentration von 2 bis 4 g/L enthält.

In einer bevorzugten Ausführungsform, umfasst das erfindungsgemässe Verfahren zusätzlich den Schritt (c) Verbinden mehrerer textiler Lagen durch Stanzen, Verschweissen, Verkleben, Vernähen oder mithilfe von Mikro- und Ultraschallwellen.

In einer weiteren Ausführungsform umfasst das erfindungsgemässe Verfahren zusätzlich den Schritt (d) Aufbringen von bioadhäsiven und/oder microadhäsiven Mitteln auf der proximalen Fläche.

Der Fachmann versteht, dass die alphabetische Aufzählung der Schritte keine Rückschlüsse auf die Reihenfolge der Schritte zulässt. So kann beispielsweise die proximale Fläche zuerst via Schritt (b) und danach via Schritt (d) beschichtet werden, in umgekehrter Reihenfolge, oder auch gleichzeitig.

Typischerweise wird das antimikrobiell und/oder antiseptisch wirksame Mittel durch Vermischen der jeweiligen Komponenten, wie oben beschrieben, vorzugsweise in den offenbarten Konzentrationen in wässriger Lösung, bereitgestellt.

Die eine oder mehrere miteinander verbundenen textilen Lagen können beispielsweise durch Eintauchen, Bestreichen, Drucken, Besprühen, Beträufeln oder durch ähnliche Methoden mit dem antimikrobiell und/oder antiseptisch wirksamen Mittel zumindest auf der proximalen Fläche beschichtet werden.

### Kurze Erläuterung der Figuren

Anhand der in den nachfolgenden Figuren gezeigten Ausführungsbeispielen und der dazugehörigen Beschreibung werden Aspekte der Erfindung näher erläutert. Es zeigen:
- Fig. 1: eine schematische Ansicht eines erfindungsgemässen Flächenprodukts zur Behandlung des Urogenitaltrakts gemäss einer Ausführungsform der Erfindung;
- Fig. 2: eine schematische Ansicht eines erfindungsgemässen Flächenprodukts zur Behandlung des Urogenitaltrakts gemäss einer weiteren Ausführungsform der Erfindung;
- Fig. 3: eine schematische Ansicht eines erfindungsgemässen Flächenprodukts zur Behandlung des Urogenitaltrakts gemäss einiger weiterer Ausführungsformen der Erfindung;
- Fig. 4: eine schematische Ansicht eines erfindungsgemässen Flächenprodukts zur Behandlung des Urogenitaltrakts gemäss einiger weiterer Ausführungsformen der Erfindung;
- Fig. 5: eine Referenzskala für die Bestimmung der PI-MA Werte.

### Wege zur Ausführung der Erfindung

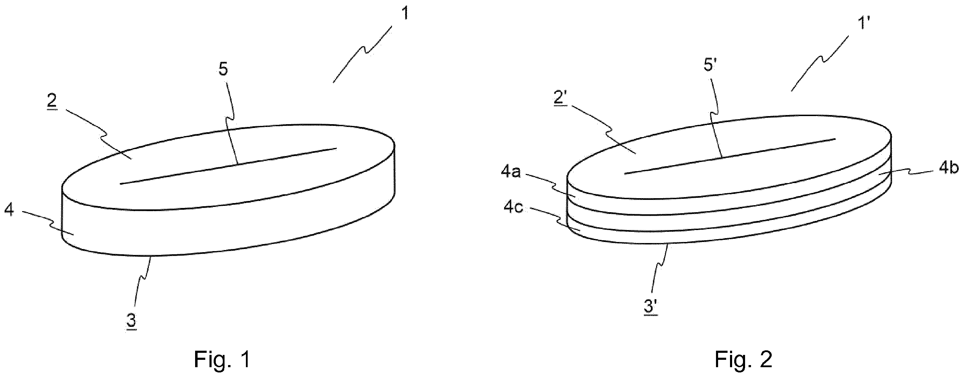

Die Figur 1 zeigt eine schematische Ansicht eines erfindungsgemässen Flächenprodukts zur Behandlung des Urogenitaltrakts 1 mit einer proximalen Fläche 2 und einer distalen Fläche 3. Das Flächenprodukt 1 umfasst eine textile Lage 4 (zur besseren Übersicht ist diese deutlich verbreitert dargestellt). Die proximale Fläche 2 ist mit einem antimikrobiell und/oder antiseptisch wirksamen Mittel beschichtet (nicht dargestellt). Im gezeigten Ausführungsbeispiel weist die proximale Fläche zusätzlich eine Steppnaht 5 auf.

In der Figur 2 ist eine weitere Ausführungsform eines Flächenprodukts zur Behandlung des Urogenitaltrakts 1' mit einer proximalen Fläche 2' und einer distalen Fläche 3' dargestellt. Das Flächenprodukt 1' umfasst drei textile Lagen 4a, 4b und 4c, welche beispielsweise durch Nähen, Kleben, Stanzen, etc. miteinander verbunden sind (zur besseren Übersicht ist diese deutlich verbreitert dargestellt). Hierbei ist es besonders vorteilhaft, wenn die mittlere Lage 4b aus einem saugfähigen Material, wie Watte oder ein Schaummaterial, besteht. Insbesondere der Lage 4c kann durch eine hydrophobe Beschichtung hydrophobiert sein. Des Weiteren weist die proximale Fläche 2' eine Steppnaht 5' in Längsrichtung auf.

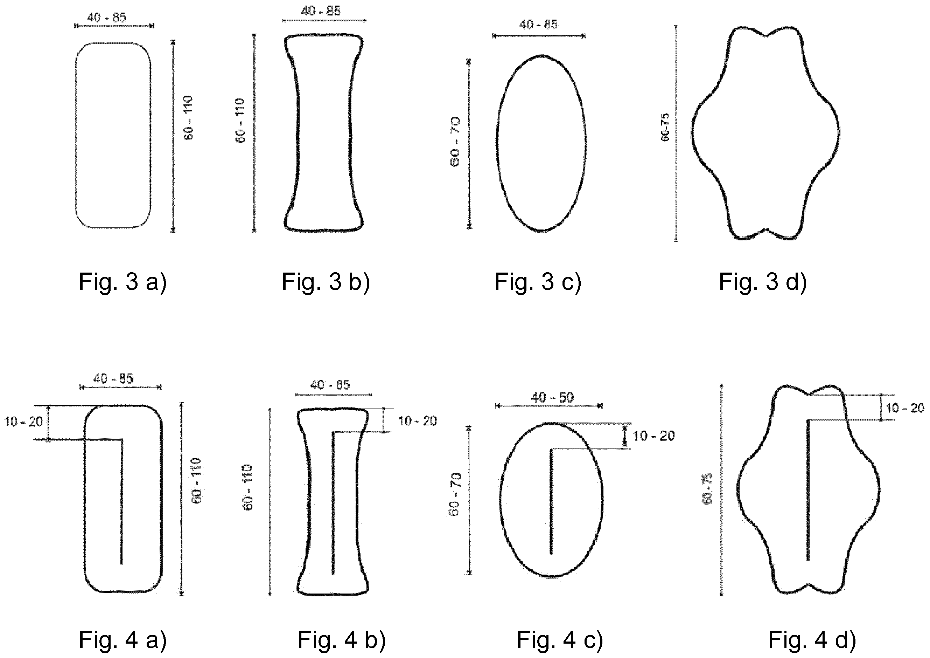

Die Figur 3 zeigt eine Aufsicht mehrerer verschiedener Ausführungsformen eines erfindungsgemässen Flächenprodukts zur Behandlung des Urogenitaltrakts . Das in Figur 3a) gezeigte Flächenprodukt weist eine längliche Form auf, welche an den Ecken abgerundet ist. In der Figur 3 b) ist die Form des erfindungsgemässen Flächenprodukts ebenfalls länglich, jedoch sind die Ränder der Längsseiten nach innen geschwungen und damit in der Aufsicht im Profil von konkaver Form. Eine solche Ausführungsform kann beispielsweise einen erhöhten Tragekomfort gewährleisten, da die Bewegungsfreiheit, insbesondere der Beine, zusätzlich erhöht wird. Die Figur 3 c) zeigt ein erfindungsgemässes Flächenprodukt, das eine ovale Form aufweist. Eine solche Ausführungsform bietet den Vorteile einer relativ grossen Fläche, wodurch der Anteil des antimikrobiell und/oder antiseptisch wirksamen Mittels erhöht werden kann. Des Weiteren ist in der Figur 3 d) eine Ausführungsform der Erfindung mit Flügeln gezeigt, bei welcher die beiden Längsseiten jeweils einen Flügel aufweisen, wie es von gängigen Hygienebinden bekannt ist. Je nach Bedarf weisen die in den Figur 3 a) bis d) eine Länge von 60 bis 110 mm und eine Breite von 40 bis 85 mm auf. Es sind jedoch auch quadratische oder kreisrunde Flächenprodukte vorgesehen oder Flächenprodukte, welche einen grösseren Bereich abdecken und dementsprechend eine Länge oder einen Durchmesser von 60 bis zu 200 mm aufweisen können.

Die Figur 4 zeigt in Aufsicht auf die jeweiligen proximalen Flächen einiger weiterer Ausführungsformen der Erfindung. Die proximale Fläche weisst dabei eine Steppnaht in Längsrichtung des Flächenprodukts auf. Eine solche Steppnaht in Längsrichtung, also im operativen Zustand in Richtung der Schamlippen, hat den Vorteil, dass die Kontaktfläche zur Haut vergrössert wird und das Flächenprodukt dicht an der Haut anliegt. Des Weiteren wird das Knicken erleichtert und ein Verrutschen während des Tragens effizienter vermieden. In den gezeigten typischen Ausführungsformen beträgt die Länge der Steppnaht etwa 2/3 der Länge oder des Durchmessers des Flächenprodukts. Beispielsweise beträgt der Abstand der Steppnaht vom Rand der des Flächenprodukts wie in den Figuren 4 a) bis d) dargestellt typischerweise 10 bis 20 mm, bei einer Länge des Flächenprodukts von 60 bis 110 mm.

Zur Bestimmung der Verträglichkeit eines Flächenprodukts zur Behandlung des Urogenitaltrakts kann der nachfolgend beschriebene Zytotoxizitätstest verwendet werden. Dieser ist angelehnt an die Norm DIN ISO 10993-5.

Es werden rekonstituierte humane 3D-EpiVaginal Modelle (beispielsweise MatTek) verwendet. Dazu wird ein Element mit 6 mm Durchmesser aus einem erfindungsgemässen Flächenprodukt topisch auf den 3D-EpiVaginal Hautmodellen appliziert und für 3 und 18 Stunden auf den Modellen inkubiert. Als Negativkontrolle werden mit sterilem Wasser behandelte Hautmodelle und als Positivkontrolle mit dem anionischen Tensid Triton X-100 (1 %) behandelte Hautmodelle verwendet. Die Vitalität der Hautmodelle wird im MTT-Assay (3-(4,5-Dimethylthiazol-2-yl)-2,5-diphenyltetrazoliumbromid) evaluiert (siehe Stockert et al. Acta Histochemica 120: 159-167 (2018), doi:10.1016/j.acthis.2018.02.005; Chacon et al. In Vitro Methods in Pharmaceutical Research, ISBN 9780080534602 and https://en.wikipedia.org/wiki/MTT_assay). Dabei wird der Vitalitätswert der Negativkontrolle auf 100% festgesetzt. Die Positivkontrolle zeigt typischerweise nach 3 h Inkubation eine Vitalität von 25% und nach 18 h eine Vitalität von weniger als 10%. Als akzeptabler Wert gilt erfindungsgemäss typischerweise eine Vitalität von >80% nach 18 h.

Die Wirksamkeit eines Flächenprodukts zur Behandlung des Urogenitaltrakts kann anhand des nachfolgend beschriebenen Tests zur Bestimmung der Pad-Inhärenten Mikrobiellen Inhibierungs-Aktivität (PI-MIATest) bestimmt werden.

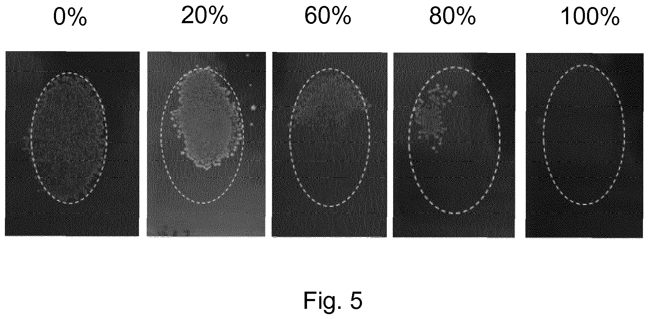

Beim PI-MIA Test wird zuerst eine frische Übernachtkultur von *E. coli* (DSM 498) herangezogen. Zudem wird eine Testlösung in sterilem Wasser mit 105 KbE/mL angesetzt (KbE = Gesamtkeimzahl). Anschliessend werden je 500 µL *E. coli* mit 105 KbE/mL auf eine sterile Testfläche aufgebracht. In der Zwischenzeit wird eine Vorrichtung zur Behandlung des Urogenitaltrakts, beispielsweise ein erfindungsgemässes Flächenprodukt welches mit 400 µL eines antimikrobiell und/oder antiseptisch wirksamen Mittels gemäss der Erfindung beschichtet wurde, sowie eine 0-Kontrolle (typischerweise eine mit Wasser beschichtete Vorrichtung) vorbereitet. Die Inkubation erfolgt dann jeweils für 60 min bei Raumtemperatur. Danach werden die Vorrichtungen (Probe und 0-Kontrolle) abgenommen und mit der bakteriell kontaminierten Kontaktseite auf eine LB-Platte (Lysogeny Broth Plate) in Kontakt gebracht und für 5 min bei Raumtemperatur inkubiert. Die Vorrichtungen werden dann abgenommen und die LB Platten für 18 Stunden bei 37 °C inkubiert. Schliesslich kann die Probe mit der 0-Kontrolle verglichen werden. Über eine Referenzskala erfolgt zudem die Bestimmung der PI-MIA Werte. Schliesslich kann die Probe mit der 0-Kontrolle verglichen werden. Über eine Referenzskala gemäss Figur 5 erfolgt zudem die Bestimmung der PI-MIA Werte.

Eine ausreichende Wirkung ist typischerweise bei 80% Wachstumsinhibierung gegeben. Die folgende Tabelle zeigt ein erfindungsgemässes Besipiel (Beispiel 3) und Vergleichsbeispiele (Beispiele 1, 2, und 4) des verwendeten antimikrobiell und/oder antiseptisch wirksamen Mittels:

| **g/l** | **Beispiel 1 [g/L]** | **Beispiel 2 [g/L]** | **Beispiel 3 [g/L]** | **Beispiel 4 [g/L]** |
|---|---|---|---|---|
| Vitamin C | 0.8806 | 0.8806 | 0.8806 | 0.8806 |
| Weinsäure | 5.2530 | 5.2530 | 3.0000 | 5.2530 |
| Zitronensäure | 6.7250 | 3.8400 | 3.8400 | 6.7250 |
| Tri-Natriumzitrat | | *4,4150* | | |
| Kupfer(II) Chlorid | 0.8524 | 0.8524 | 0.8524 | 0.8524 |
| ZnSO₄ | | | | |
| FeSO₄ | | | | |
| Natriumhydroxid | | | | 2-3g/L |
| Natriumdodecylsulfat | 2.5000 | 2.5000 | 2.5000 | 2.5000 |
| | | | | |
| pH-Wert | ∼ 1.9 | ∼2.9 | ∼2.3 | ∼2.9 |
| | | | | |
| PI-MIA Test | - | not 100% | ok | ok |
| Zytotoxizität/Zelle Vitalität nach 3 h | 64.9 | 61.1 | 87.1 | 74.6 |
| Zytotoxizität/Zelle Vitalität nach 18 h | 54.2 | 41.7 | 81.7 | 67.5 |

Es zeigt sich, dass sich die Verwendung von Weinsäure in einer Konzentration von maximal 6 g/L im Allgemeinen vorteilhaft auf die Vitalität auswirkt. Hierbei das Ersetzen von CuCI durch FeSO₄ (Konzentration von 0.9632 g/L) und ZnSO₄ (Konzentration 1.0235 g/L) vergleichbare Werte.

## Patentansprüche

1. Flächenprodukt (1, 1') zur Behandlung des Urogenitaltrakts mit einer proximalen Fläche (2, 2') und einer distalen Fläche (3, 3'), wobei das Flächenprodukt mindestens eine textile Lage (4) umfasst und wobei die proximale Fläche mit einem antimikrobiell und/oder antiseptisch wirksamen Mittel beschichtet ist, und wobei das Flächenprodukt im hier beschriebenen Zytotoxizitätstest eine Vitalität von > 80% nach 18 h Inkubationszeit und im hier beschriebenen PI-MIA Test eine Wirksamkeit von > 80 % aufweist, **dadurch gekennzeichnet, dass** das antimikrobiell und/oder antiseptisch wirksame Mittel Weinsäure in einer Konzentration von 2 bis 4 g/L, enthält.

2. Flächenprodukt (1, 1') nach Anspruch 1, wobei die proximale Fläche (2, 2') in der Mitte in Längsrichtung eine Steppnaht (5) zur Erleichterung des Knickens des Flächenprodukts aufweist.

3. Flächenprodukt (1, 1') nach einem der vorherigen Ansprüche, wobei die proximale Fläche (2, 2') in Streifen oder punktuell zur besseren Fixierung auf der Haut mit bioadhäsiven und/oder mucoadhäsiven Mitteln beschichtet ist.

4. Flächenprodukt (1, 1') nach einem der vorherigen Ansprüche, wobei das antimikrobiell und/oder antiseptisch wirksame Mittel Tenside, quaternäre Ammoniumverbindungen, Biguanide, organische Säuren, Metallionen wie Kupfer, Zink, Eisen, Silber, Ruthenium, Rhodium, Jodverbindungen, Aldehyde und Mischungen derselben umfasst.

5. Flächenprodukt (1, 1') nach einem der vorherigen Ansprüche, wobei das antimikrobiell und/oder antiseptisch wirksame Mittel umfasst: mindestens ein Vitamin ausgewählt aus Vitamin A, B, C, E; mindestens ein Metallion der achten, elften oder zwölften Gruppe des Periodensystems; mindestens eine aliphatische oder aromatische organische Säure oder Derivate derselben; mindestens eine oberflächenaktive Verbindung; optional Basen und/oder Puffersysteme zur Einstellung des pH Werts.

6. Flächenprodukt (1, 1') nach einem der vorherigen Ansprüche, wobei das antimikrobiell und/oder antiseptisch wirksame Mittel mindestens einen Gelbildner umfasst.

7. Flächenprodukt (1, 1') nach Anspruch 6, wobei der mindestens eine Gelbildner ausgewählt wird aus der Gruppe der Polysacchariden, vorzugsweise Agarose, Carrageenan, Pektin, Xanthan, Alginsäure und Alginate, Siliziumdioxid, Polyvinylpyrrolidone, Polyvinylalkohole, Polymethacrylate, vorzugsweise Poly(hydroxy methacrylate), Poly(N-isopropyl acrylamide) Polyacrylsäuren oder Mischungen derselben.

8. Flächenprodukt (1, 1') nach einem der Ansprüche 4 bis 7, wobei das antimikrobiell und/oder antiseptisch wirksame Mittel als Vitamin Ascorbinsäure, als Metallion Kupfer, als organische Säure Zitronen- und/oder Weinsäure, als Tensid Laurylsulfate, insbesondere Natriumlaurylsuflat, und optional als Gelbildner Agarose oder Carrageenan enthält.

9. Flächenprodukt (1, 1') nach einem der Ansprüche 4 bis 8, wobei die Konzentration
des mindestens einen Vitamins 0.5 bis 1.5 g/L beträgt; und /oder
der mindestens einen aliphatischen oder aromatischen Säure 2 bis 6.5 g/L beträgt; und/oder
der Metallionen 0.5 bis 1.5 g/L beträgt; und/oder
der Tenside 1.5 bis 10 g/L, bevorzugt 2 bis 8 g/L, besonders bevorzugt 2.5 bis 6.6 g/L beträgt; und/oder
des mindestens einen Gelbildners 2.5 bis 45 g/L, bevorzugt 5 bis 35 g/L, besonders bevorzugt 7.5 g/L bis 25 g/L beträgt.

10. Flächenprodukt (1, 1') nach einem der vorherigen Ansprüche, wobei das antimikrobiell und/oder antiseptisch wirksame Mittel einen pH-Wert zwischen 2.3 bis 3.1 aufweist.

11. Flächenprodukt (1, 1') nach einem der vorherigen Ansprüche, wobei das Flächenprodukt eine Form aufweist, die derart ausgestaltet ist, dass das Flächenprodukt interlabial platzierbar oder tragbar ist.

12. Flächenprodukt (1, 1') nach einem der vorherigen Ansprüche zur Anwendung in der Therapie oder Prophylaxe.

13. Verfahren zur Herstellung eines Flächenprodukts zur Behandlung des Urogenitaltrakts nach Anspruch 1, umfassend die Schritte:
(a) Bereitstellen mindestens einer textilen Lage;
(b) Beschichtung einer proximalen Fläche mit einem antimikrobiell und/oder antiseptisch wirksamen Mittel, sodass das Flächenprodukt im hier beschriebenen Zytotoxizitätstest eine Vitalität von > 80% nach 18 h Inkubationszeit und im hier beschriebenen PI-MIA Test eine Wirksamkeit von > 80 % aufweist **dadurch gekennzeichnet, dass** das antimikrobiell und/oder antiseptisch wirksame Mittel Weinsäure in einer Konzentration von 2 bis 4 g/L, enthält.

14. Verfahren nach Anspruch 13 zusätzlich umfassend den Schritt:
(c) Verbinden mehrerer textilen Lagen durch Stanzen, Verschweissen, Verkleben, Vernähen oder mithilfe von Mikro- oder Ultraschallwellen.

15. Verfahren nach einem der Ansprüche 13 oder 14 zusätzlich umfassend den Schritt:
(d) Aufbringen von bioadhäsiven und/oder mucoadhäsiven Mitteln auf der proximalen Fläche.

## Claims

1. Planar product (1, 1') for treatment of the urogenital tract having a proximal face (2, 2') and a distal face (3, 3'), wherein the planar product includes at least one textile layer (4) and wherein the proximal face is coated with an antimicrobially and/or antiseptically effective agent, and wherein the planar product has a vitality of > 80% after 18 h of incubation time in the cytotoxicity test described here and an efficacy of > 80% in the PI-MIA test described here, **characterized in that** the antimicrobially and/or antiseptically effective agent contains tartaric acid in a concentration of 2 to 4 g/L.

2. Planar product (1, 1') according to Claim 1, wherein the proximal face (2, 2') comprises a top stitching (5) in the center in the longitudinal direction for facilitation of bending of the planar product.

3. Planar product (1, 1') according to either of the preceding claims, wherein the proximal face (2, 2') is coated with bioadhesive and/or mucoadhesive agents in stripes or in a punctiform manner for better fixation on the skin.

4. Planar product (1, 1') according to any of the preceding claims, wherein the antimicrobially and/or antiseptically effective agent comprises surfactants, quaternary ammonium compounds, biguanides, organic acids, metal ions such as copper, zinc, iron, silver, ruthenium, rhodium, iodine compounds, aldehydes and mixtures thereof.

5. Planar product (1, 1') according to any of the preceding claims, wherein the antimicrobially and/or antiseptically effective agent comprises: at least one vitamin selected from vitamin A, B, C, E; at least one metal ion of group eight, eleven or twelve of the periodic table; at least one aliphatic or aromatic organic acid or derivatives thereof; at least one surface-active compound; optionally bases and/or buffer systems for pH adjustment.

6. Planar product (1, 1') according to any of the preceding claims, wherein the antimicrobially and/or antiseptically effective agent includes at least one gel former.

7. Planar product (1, 1') according to Claim 6, wherein the at least one gel former is selected from the group of polysaccharides, preferably agarose, carrageenan, pectin, xanthan gum, alginic acid and alginates, silicon dioxide, polyvinylpyrrolidones, polyvinyl alcohols, polymethacrylates, preferably poly(hydroxy methacrylates), poly(N-isopropylacrylamides), polyacrylic acids or mixtures thereof.

8. Planar product (1, 1') according to any of Claims 4 to 7, wherein the antimicrobially and/or antiseptically effective agent contains ascorbic acid as vitamin, copper as metal ion, citric and/or tartaric acid as organic acid, lauryl sulfates, especially sodium lauryl sulfate, as surfactant and optionally agarose or carrageenan as gel former.

9. Planar product (1, 1') according to any of Claims 4 to 8, wherein the concentration
of the at least one vitamin is 0.5 to 1.5 g/L; and/or
that of the at least one aliphatic or aromatic acid is 2 to 6.5 g/L; and/or
that of the metal ions is 0.5 to 1.5 g/L; and/or
that of the surfactants is 1.5 to 10 g/L, preferably 2 to 8 g/L, particularly preferably 2.5 to 6.6 g/L; and/or that of the at least one gel former is 2.5 to 45 g/L, preferably 5 to 35 g/L, particularly preferably 7.5 g/L to 25 g/L.

10. Planar product (1, 1') according to any of the preceding claims, wherein the antimicrobially and/or antiseptically effective agent has a pH between 2.3 to 3.1.

11. Planar product (1, 1') according to any of the preceding claims, wherein the planar product has a shape which is configured such that the planar product is placeable or wearable interlabially.

12. Planar product (1, 1') according to any of the preceding claims for use in therapy or prophylaxis.

13. Method for producing a planar product for treatment of the urogenital tract according to Claim 1, including the steps of:
(a) providing at least one textile layer;
(b) coating a proximal face with an antimicrobially and/or antiseptically effective agent, such that the planar product has a vitality of > 80% after 18 h of incubation time in the cytotoxicity test described here and an efficacy of > 80% in the PI-MIA assay described here, **characterized in that** the antimicrobially and/or antiseptically effective agent contains tartaric acid in a concentration of 2 to 4 g/L.

14. Method according to Claim 13, additionally including the step of:
(c) joining multiple textile layers by stamping, welding, bonding, sewing or with the aid of microwaves or ultrasonic waves.

15. Method according to either of Claims 13 and 14, additionally including the step of:
(d) applying bioadhesive and/or mucoadhesive agents on the proximal face.

## Revendications

1. Produit plat (1, 1') pour le traitement du tractus génito-urinaire, ayant une surface proximale (2, 2') et une surface distale (3, 3'), le produit plat comprenant au moins une couche textile (4), et la surface proximale étant revêtue d'un agent à activité antimicrobienne et/ou antiseptique, et le produit plat présentant, dans l'essai de cytotoxicité décrit ici, une vitalité > 80 % après un temps d'incubation de 18 heures et, dans l'essai PI-MIA décrit ici, une activité > 80 %, **caractérisé en ce que** l'agent à activité antimicrobienne et/ou antiseptique contient de l'acide tartrique à une concentration de 2 à 4 g/l.

2. Produit plat (1, 1') selon la revendication 1, dans lequel la surface proximale (2, 2') présente en son milieu dans la direction longitudinale une surpiqûre (5) destinée à faciliter le repliement du produit plat.

3. Produit plat (1, 1') selon l'une des revendications précédentes, dans lequel la surface proximale (2, 2') est, en bandes ou ponctuellement pour améliorer la fixation sur la peau, revêtue d'agents bioadhésifs et/ou mucoadhésifs.

4. Produit plat (1, 1') selon l'une des revendications précédentes, dans lequel l'agent à activité antimicrobienne et/ou antiseptique comprend des tensioactifs, des composés de l'ammonium quaternaire, des biguanides, des acides organiques, des ions métalliques tels que des ions cuivre, zinc, fer, argent, ruthénium, rhodium, des composés de l'iode, des aldéhydes et des mélanges de ceux-ci.

5. Produit plat (1, 1') selon l'une des revendications précédentes, dans lequel l'agent à activité antimicrobienne et/ou antiseptique comprend au moins une vitamine choisie parmi la vitamine A, B, C, E ; au moins un ion métallique du huitième, du onzième ou du douzième groupe du système périodique ; au moins un acide organique aliphatique ou aromatique ou des dérivés de celui-ci ; au moins un composé tensioactif, éventuellement des bases et/ou des systèmes tampons pour ajuster le pH.

6. Produit plat (1, 1') selon l'une des revendications précédentes, dans lequel l'agent à activité antimicrobienne et/ou antiseptique comprend au moins un gélifiant.

7. Produit plat (1, 1') selon la revendication 6, dans lequel l'au moins un gélifiant est choisi dans le groupe des polysaccharides, de préférence l'agarose, le carragénane, la pectine, le xanthane, l'acide alginique et les alginates, le dioxyde de silicium, les polyvinylpyrrolidones, les poly(alcools vinyliques), les polyméthacrylates, de préférence les poly(hydroxy méthacrylates), les poly(N-isopropyl acrylamides), les poly(acides acryliques) ou les mélanges de ceux-ci.

8. Produit plat (1, 1') selon l'une des revendications 4 à 7, dans lequel l'agent à activité antimicrobienne et/ou antiseptique contient en tant que vitamine de l'acide ascorbique, en tant qu'ion métallique du cuivre, en tant qu'acide organique de l'acide citrique et/ou tartrique, en tant que tensioactif des laurylsulfates, en particulier du laurylsulfate de sodium, et éventuellement en tant que gélifiant de l'agarose ou du carragénane.

9. Produit plat (1, 1') selon l'une des revendications 4 à 8, dans lequel la concentration
de l'au moins une vitamine est de 0,5 à 1,5 g/l ; et/ou
de l'au moins un acide aliphatique ou aromatique est de 2 à 6,5 g/l ; et/ou
des ions métalliques est de 0,5 à 1,5 g/l ; et/ou
des tensioactifs est de 1,5 à 10 g/l, de préférence de 2 à 8 g/l, d'une manière particulièrement préférée de 2,5 à 6,6 g/l ; et/ou
de l'au moins un gélifiant est de 2,5 à 45 g/l, de préférence de 5 à 35 g/l, d'une manière particulièrement préférée de 7,5 g/l à 25 g/l.

10. Produit plat (1, 1') selon l'une des revendications précédentes, dans lequel l'agent à activité antimicrobienne et/ou antiseptique présente un pH entre 2,3 et 3,1.

11. Produit plat (1, 1') selon l'une des revendications précédentes, le produit plat ayant une forme qui est conçue pour que le produit plat puisse être placé ou porté en position interlabiale.

12. Produit plat (1, 1') selon l'une des revendications précédentes, pour utilisation en thérapie ou prophylaxie.

13. Procédé de fabrication d'un produit plat pour le traitement du tractus génito-urinaire selon la revendication 1, comprenant les étapes suivantes :
(a) fourniture d'au moins une couche textile ;
(b) application d'un agent à activité antimicrobienne et/ou antiseptique sur une surface proximale, de façon que le produit plat présente dans l'essai de cytotoxicité décrit dans l'invention une vitalité > 80 % après un temps d'incubation de 18 heures et, dans l'essai PI-MIA décrit dans l'invention, une activité > 80 %, **caractérisé en ce que** l'agent à activité antimicrobienne et/ou antiseptique contient de l'acide tartrique à une concentration de 2 à 4 g/l.

14. Procédé selon la revendication 13, comprenant en outre l'étape suivante :
(c) assemblage de plusieurs couches textiles par poinçonnage, soudage, collage, couture, ou à l'aide de microondes ou d'ultrasons.

15. Procédé selon l'une des revendications 13 ou 14, comprenant en outre l'étape suivante :
(d) application, sur la surface proximale, d'agents bioadhésifs et/ou mucoadhésifs.
